(19) Europäisches Patentamt
European Patent Office
Office européen des brevets

(11) Veröffentlichungsnummer : **0 071 835**
**B1**

(12) # EUROPÄISCHE PATENTSCHRIFT

(45) Veröffentlichungstag der Patentschrift : **07.11.84**

(51) Int. Cl.³ : **C 07 C125/063**

(21) Anmeldenummer : **82106635.4**

(22) Anmeldetag : **23.07.82**

(54) **Verfahren zur Herstellung von Urethanen.**

(30) Priorität : **04.08.81 DE 3130843**

(43) Veröffentlichungstag der Anmeldung : **16.02.83 Patentblatt 83/07**

(45) Bekanntmachung des Hinweises auf die Patenterteilung : **07.11.84 Patentblatt 84/45**

(84) Benannte Vertragsstaaten : **BE DE FR GB IT NL**

(56) Entgegenhaltungen : **US-A- 4 266 070**

(73) Patentinhaber : **BAYER AG**
**Konzernverwaltung RP Patentabteilung**
**D-5090 Leverkusen 1 Bayerwerk (DE)**

(72) Erfinder : **Stammann, Günter, Dr.**
**Silesiusstrasse 78**
**D-5000 Koeln 80 (DE)**
Erfinder : **Becker, Robert, Dr.**
**Martin-Heidegger-Strasse 8**
**D-5090 Leverkusen 3 (DE)**
Erfinder : **Grolig, Johann, Dr.**
**Heinrich-Luebke-Strasse 22**
**D-5090 Leverkusen 1 (DE)**
Erfinder : **Waldmann, Helmut, Dr.**
**Henry-T.-von-Boettingerstrasse 15**
**D-5090 Leverkusen 1 (DE)**

**EP 0 071 835 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft ein neues Verfahren zur Herstellung von Urethanen (Carbamidsäureestern oder Carbamaten) durch Umsetzung von Harnstoffen mit organischen Hydroxylverbindungen und Kohlenmonoxid in Gegenwart von molekularem Sauerstoff und eines mindestens ein Edelmetall oder mindestens eine Edelmetallverbindung aufweisenden Katalysatorsystems, wobei man die Umsetzung in Gegenwart von chinoiden Verbindungen oder von in chinoide Verbindungen überführbaren Verbindungen durchführt.

Organische Isocyanate werden großtechnisch im allgemeinen durch Umsetzung der entsprechenden Amine mit Phosgen hergestellt. Wegen des hohen Chlorbedarfs und der damit verbundenen hohen Energiekosten für die Phosgenherstellung ist man seit geraumer Zeit bestrebt, einen großtechnisch gangbaren Weg zu organischen Isocyanaten aufzufinden, bei welchen sich die Verwendung von Phosgen erübrigt.

Ein derartiger Weg besteht z. B. in der phosgenfreien Herstellung von niedermolekularen Urethanen und deren anschließende thermische Spaltung. Bezüglich der Herstellung von derartigen, spaltbaren Urethanen sind bereits mehrere Wege vorgeschlagen worden. Die DE-OS 2 908 250, DE-OS 2 908 251, DE-OS 2 910 132, US-PS 4 260 781 und US-PS 4 266 070 beschreiben beispielsweise die Herstellung von Urethanen aus primären Aminen, Kohlenoxid und organischen Hydroxylverbindungen in Gegenwart von Oxidationsmitteln, wobei gemäß US-PS 4 266 070 als Oxidationsmittel Chinone unter Abwesenheit von Sauerstoff und als Katalysatoren schwer handhabbare Carbonylverbindungen zum Einsatz gelangen.

Ein weiterer Weg zu den genannten, spaltbaren Urethanen besteht in der Umsetzung von N-N'-disubstituierten Harnstoffen mit Hydroxylgruppen aufweisenden Verbindungen und Kohlenmonoxid in Gegenwart geeigneter Oxidationsmittel. Gemäß DE-OS 2 908 252 werden für diese Reaktion als Oxidationsmittel Nitroverbindungen oder auch molekularer Sauerstoff, allein oder in Kombination mit Nitroverbindungen, eingesetzt. Hierbei verläuft die Reaktion nach folgender allgemeiner Gleichung (1)

Gl. (1)

$$2\ R^1\text{-NH-}\overset{\overset{O}{\|}}{C}\text{-NH-}R^2 + R^3\text{-}NO_2 + 5\ R^4\text{-OH} + 3\ CO \longrightarrow$$

$$2\ R^1\text{-NH-}\overset{\overset{O}{\|}}{C}\text{-O}R^4 + 2\ R^2\text{-NH-}\overset{\overset{O}{\|}}{C}\text{-O}R^4 + R^3\text{-NH-}\overset{\overset{O}{\|}}{C}\text{-O}R^4 + 2\ H_2O$$

mit $R^1$ bis $R^4$ = Alkyl oder Aryl.

Bei Verwendung von molekularem Sauerstoff als Oxidationsmittel verläuft die Umsetzung nach folgender Gleichung (2):

Gl. (2)

$$R^1\text{-NH-}\overset{\overset{O}{\|}}{C}\text{-NH-}R^2 + 2\ R^4\text{-OH} + CO + 1/2\ O_2 \longrightarrow$$

$$R^1\text{-NH-}\overset{\overset{O}{\|}}{C}\text{-O}R^4 + R^2\text{-NH-}\overset{\overset{O}{\|}}{C}\text{-O}R^4 + H_2O$$

Nachteilig bei der Verwendung von Nitroverbindungen als Oxidationsmittel ist hierbei, daß ein Gemisch von Urethanen entsteht, welches z. B. durch Destillation aufgetrennt werden muß. Diese Methode dürfte nur sinnvoll sein, wenn die Substituenten $R^1$ bis $R^3$ gleich sind, da nur so ein zwangsweise anfallendes Nebenprodukt vermieden werden kann. Nur in Sonderfällen sind jedoch die entsprechenden Harnstoffe und Nitroverbindungen gleichermaßen technisch leicht verfügbar. Unabhängig von der Art des Oxidationsmittels ist ein weiterer Nachteil des Verfahrens gemäß DE-OS 2 980 252 in der zwingend erforderlichen Mitverwendung relativ hoher Mengen korrodierend wirkender, als Cokatalysatoren erforderlicher anorganischer oder organischer Halogenide zu sehen, wobei neben der korrodierenden Wirkung auch die weitgehende Unlöslichkeit dieser Cokatalysatoren die großtechnische Durchführbarkeit des beschriebenen Verfahrens negativ beeinträchtigt.

Es war daher die Aufgabe der Erfindung, ein verbessertes Verfahren zur Herstellung von Urethanen aus Harnstoffen, Hydroxylgruppen aufweisenden organischen Verbindungen und Kohlenmonoxid in Gegenwart von molekularem Sauerstoff als Oxidationsmittel zur Verfügung zu stellen, bei welchem auf die Verwendung von weitgehend unlöslichen bzw. von korrodierend wirkenden Katalysatorkomponenten verzichtet werden kann, bzw. bei welchen der Anteil derartiger Komponenten im Reaktionsgemisch ganz wesentlich reduziert ist.

Diese Aufgabe konnte überraschenderweise durch das nachstehend näher erläuterte erfindungsgemäße Verfahren gelöst werden, bei welchem die geschilderten, durch das Cokatalysatorsystem verursachten Probleme weitgehend entfallen.

Gegenstand der Erfindung ist ein Verfahren zur Herstellung von Urethanen durch Umsetzung von

a) unsubstituierten oder substituierten Harnstoffen, welche jedoch noch mindestens ein, an ein Harnstoff-Stickstoffatom gebundenes Wasserstoffatom aufweisen, mit
b) mindestens eine Hydroxylgruppe enthaltenden organischen Verbindungen und
c) Kohlenmonoxid in Gegenwart von
d) molekularem Sauerstoff und
e) einem mindestens ein Edelmetall und/oder eine Edelmetallverbindung der achten Nebengruppe des Periodensystems der Elemente enthaltenden Katalysatorsystem,

dadurch gekennzeichnet, daß man die Umsetzung in Gegenwart von Katalysatorsystemen e) durchführt, die als weitere Komponente mindestens eine oxidierend wirkende chinoide Verbindung und/oder mindestens eine Verbindung enthalten, welche unter den Reaktionsbedingungen in eine oxidierend wirkende chinoide Verbindung überführt werden kann.

Ausgangsmaterialien für das erfindungsgemäße Verfahren sind

a) Harnstoff oder substituierte Harnstoffe, welche mindestens ein, an ein Harnstoff-Stickstoffatom gebundenes Wasserstoffatom aufweisen,
b) Hydroxylgruppen enthaltende organische Verbindungen und
c) Kohlenmonoxid.

Das erfindungsgemäße Verfahren wird in Gegenwart von

d) molekularem Sauerstoff als Oxidationsmittel und von
e) einem, mindestens ein Edelmetall und/oder eine Edelmetallverbindung enthaltenden Katalysatorsystem

durchgeführt.

Geeignete Harnstoffe a) sind beliebige organische Verbindungen, die mindestens eine Struktureinheit der Formel

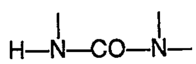

aufweisen, wobei die freien Valenzen durch Wasserstoffatome oder gegebenenfalls substituierte, unter den Reaktionsbedingungen vorzugsweise inerte organische Substituenten abgesättigt sind.

Beispiele derartiger Harnstoffe sind Harnstoff selbst oder substituierte Harnstoffe der Formeln (I), (II), (III)

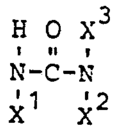

(I)

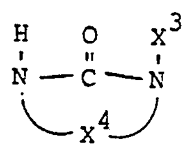

(II)

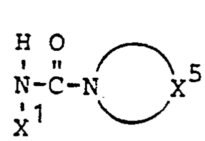

(III)

Die Substituenten $X^1$ bis $X^3$ können gleich oder verschieden sein und für Wasserstoff oder beliebige, gegebenenfalls substituierte, unter den Reaktionsbedingungen inerte organische Reste, insbesondere aliphatische Kohlenwasserstoffreste mit 1 bis 4 Kohlenstoffatomen oder aromatische Kohlenwasserstoffreste mit 6 bis 10 Kohlenstoffatomen stehen. Der zweiwertige Substituent $X^4$ bildet mit den beiden Stickstoffatomen der Harnstoffgruppierung einen mindestens 5-gliedrigen heterocyclischen Ring und weist mindestens ein, vorzugsweise 2 bis 6 innerhalb des Rings angeordnete Kohlenstoffatome und gegebenenfalls zusätzlich im Ring angeordnete Heteroatome auf. Der zweiwertige Substituent $X^5$ bildet mit einem der Stickstoffatome der Harnstoffgruppierung als Ringglied einen heterocyclischen Ring mit vorzugsweise 5 bis 6 Ringgliedern und weist neben mindestens zwei innerhalb des Rings angeordneten Kohlenstoffatomen gegebenenfalls weitere innerhalb des Rings angeordnete Heteroatome auf.

Alle Substituenten $X^1$ bis $X^5$ können substituiert oder durch difunktionelle Gruppen unterbrochen sein. Geeignete Substituenten bzw. difunktionelle Gruppen sind z. B. Halogenatome, Oxogruppen, Sulfoxidgruppen, Sulfongruppen, Ethergruppen, Carbonsäureester- oder Nitrogruppen. Als weitere funktionelle Gruppen sind auch Harnstoffgruppen möglich, d. h. ein Molekül der Ausgangsverbindung a) kann mehr als eine Harnstoffgruppierung enthalten. Entsprechen diese Harnstoffgruppierungen den Typen I bis III, wo werden sie ebenfalls erfindungsgemäß zu Urethangruppen umgesetzt. Enthält das Ausgangsmaterial a) eine oder mehrere Aminogruppen, so werden diese unter den Reaktionsbedingungen gemäß DE-OS 3 046 982 ebenfalls in Urethangruppen überführt. Die Molekulargewichte der geeigneten Ausgangsmaterialien a) reichen von 60 bis ca. 600.

Vorzugsweise werden als Komponente a) Harnstoffe der Formeln (I) und (II), die symmetrisch substituiert sind, eingesetzt. Besonders bevorzugt kommen N,N'-disubstituierte Harnstoffe der Formel (I) zum Einsatz, in denen beide Substituenten für gleiche Reste, insbesondere für $C_1$-$C_4$-Alkylreste oder einen Phenylrest stehen. Gut geeignet ist auch unsubstituierter Harnstoff.

Als Beispiele geeigneter Ausgangsmaterialien a) werden genannt : Harnstoff, N-tert.-butylharnstoff, N,N-Dimethylharnstoff, N,N'-Dimethylharnstoff, N-Phenyl-N,N'-dimethylharnstoff, Tris-(2-chlorphenyl)-harnstoff, 6-Nitro-1-naphthylharnstoff, 3-Amino-4-methylphenylharnstoff, Imidazolidinon-2, 1,4-Butylen-bis-harnstoff, N,N-Pentamethylenharnstoff, N,N'-Bis-(2-furanyl)-harnstoff, 1-Oxa-6,8-diazacyclododecanon-7 und N,N'-2,2'-biphenylenharnstoff.

Bevorzugte Ausgangsmaterialien a) sind beispielsweise Harnstoff, N,N'-Dimethylharnstoff, N,N'-Dicyclohexylharnstoff, N,N'-Diphenylharnstoff, N,N'-Bis-(p-tolyl)-harnstoff, N,N'-Bis-(2-aminophenyl)-harnstoff, N,N'-Bis-(4-nitrophenyl)-harnstoff und N,N'-Bis-(4-chlorphenylharnstoff).

Besonders bevorzugte Ausgangsverbindungen a) sind N,N'-Dimethylharnstoff und N,N'-Diphenylharnstoff.

Als Ausgangsmaterial b) sind beliebige Hydroxylgruppen aufweisende organische Verbindungen, insbesondere ein- oder mehrwertige Alkohole, oder ein- oder mehrwertige Phenole, geeignet. Vorzugsweise werden beim erfindungsgemäßen Verfahren gesättigte, einwertige, aliphatische Alkohole des Molekulargewichtsbereichs 32 bis 300 mit primären Hydroxylgruppen oder einwertige Phenole eines max. Molekulargewichts von 300 eingesetzt.

Bei den Alkoholen kann es sich um beliebige lineare oder verzweigte ein- oder mehrwertige Alkanole oder Alkenole, um beliebige ein- oder mehrwertige Cycloalkanole, Cycloalkenole oder Aralkylalkohole handeln. Auch beliebige inerte Substituenten aufweisende Alkohole sind geeignet. Geeignete Substituenten sind z. B. Halogenatome, Sulfoxidgruppen, Sulfongruppen, Nitrogruppen, Carbonyl- oder Carbonsäureestergruppen. Auch Etherbrücken aufweisende Alkohole sind grundsätzlich geeignet. Beispiele geeigneter Alkohole sind : Methanol, Ethanol, n-Propanol, Isopropanol, n-Butanol, n-Pentanol, n-Hexanol, Cyclohexanol, Benzylalkohol, Chlorethanol, Ethylenglykol, Diethylenglykol, Propylenglykol, Dipropylenglykol, Glycerin, Hexantriol oder Trimethylolpropan. Besonders bevorzugt werden einwertige aliphatische Alkohole mit 1 bis 6 Kohlenstoffatomen eingesetzt.

Geeignete Phenole sind beispielsweise Phenol, 2-Isopropoxyphenol, 7-Hydroxy-2,2-dimethyl-2,3-dihydrobenzofuran, die isomeren Chlorphenole, Kresole, Ethylphenole, Propylphenole, Butylphenole oder höhere Alkylphenole, Brenzcatechin, 4,4'-Dihydroxydiphenylmethan, Bisphenol A, Anthranol, Phenanthrol, Pyrogallol, Phloroglucin oder 8-Chinolinol. Bevorzugt werden einkernige Phenole mit bis zu 12 C-Atomen und zweikernige Phenole mit bis zu 18 C-Atomen eingesetzt.

Bei der Durchführung der erfindungsgemäßen Verfahrens werden die organische Hydroxylgruppen enthaltenden Ausgangsstoffe b) im allgemeinen in solchen Mengen eingesetzt, daß für jedes Mol an im Reaktionsgemisch vorliegenden Harnstoffgruppierungen der Ausgangsstoffe a) 2 bis 200, vorzugsweise 2 bis 100 Mol an Hydroxylgruppen vorliegen. Da im allgemeinen relativ billige und unter den Reaktionsbedingungen flüssige Hydroxylverbindungen b) zum Einsatz gelangen, werden diese vorzugsweise im Überschuß, innerhalb der genannten Bereiche, eingesetzt. Die überschüssigen Mengen der Hydroxylverbindungen dienen dann als Reaktionsmedium (Lösungsmittel).

Als weiterer Reaktionspartner c) kommt beim erfindungsgemäßen Verfahren Kohlenmonoxid zum Einsatz. Dieses Ausgangsmaterial wird im allgemeinen in einer Menge eingesetzt, die 0,5 bis 30 Mol Kohlenmonoxid pro Mol zu erzeugtendem Urethan entspricht, d. h. es kommen für jedes Mol an im Reaktionsgemisch vorliegender Harnstoffgruppierung des Einsatzstoffes a) im allgemeinen 0,5 bis 30 Mol Kohlenmonoxid zum Einsatz. Sollen gleichzeitig primäre Aminogruppen gemäß DE-OS 3 046 982 zu Urethangruppen umgesetzt werden, so muß pro Mol umzusetzender Aminogruppe mindestens ein weiteres Mol Kohlenmonoxid angeboten werden.

Als Oxidationsmittel d) kann molekularer Sauerstoff in reiner Form oder in Form von Gemischen mit Inertgas, wie Stickstoff oder Kohlendioxid, insbesondere in Form von Luft, eingesetzt werden. In Gegenwart von molekularem Sauerstoff erfolgt die Oxycarbonylierung z. B. nach der Reaktionsgleichung (2) oder im Falle von symmetrisch substituierten Einsatzstoffen a) nach folgender beispielhafter Gleichung (3) :

Gl. (3)
$$R^1-\overset{H}{N}-\overset{O}{\overset{\|}{C}}-\overset{H}{N}-R^1 + 2\ R^4-OH + CO + \frac{1}{2}\ O_2 \longrightarrow 2\ R^1-\overset{H}{N}-\overset{O}{\overset{\|}{C}}-OR^4 + H_2O$$

d. h., je gebildeter Urethangruppe wird 1/2 Mol Kohlenmonoxid und 1/4 Mol molekularer Sauerstoff benötigt. Im allgemeinen wird molekularer Sauerstoff bezüglich der umzusetzenden Harnstoffgruppen in etwa stöchiometrischer bis etwa fünffacher Überschuß-Menge eingesetzt. Bei Verwendung von gegenüber Oxidationsmitteln empfindlichen Einsatzstoffen b) kann es oft auch zweckmäßig sein, den als Oxidationsmittel d) verwendeten Sauerstoff im Unterschuß, bezogen auf die Harnstoffgruppen der Komponente a), einzusetzen. Dies bedeutet, daß es durchaus zweckmäßig sein kann, den Sauerstoff

lediglich in einer Menge einzusetzen, die 60 bis 100 % der gemäß obiger Gleichung erforderlichen, äquivalenten Menge entspricht, da bei Verwendung von oxidationsempfindlichen Einsatzstoffen b) die durch unerwünschte Oxidationsreaktionen hervorgerufenen Ausbeuteverluste schwerer ins Gewicht fallen können als die durch Verwendung unterschüssiger Mengen Oxidationsmittel d) hervorgerufenen Ausbeuteverluste. Bei Verwendung von unterschüssigen Mengen an Sauerstoff, entstehen im übrigen Reaktionsgemische, in denen leicht wiedergewinnbare Ausgangsstoffe vorliegen, welche erneut für das erfindungsgemäße Verfahren eingesetzt werden können. Durch unerwünschte Oxidationsreaktionen vernichteter Alkohol kann jedoch nicht wiedergewonnen werden.

Als Oxidationsmittel d) sind prinzipiell auch beliebige, oxidierend wirkende anorganische, weitgehend ionische, insbesondere salzartige Verbindungen von Metallen in höheren Wertigkeitsstufen geeignet, die in mehreren Wertigkeitsstufen vorliegen können. Auch organische Oxidationsmittel, insbesondere Chinone sind prinzipiell geeignet. Alle diese Oxidationsmittel sind jedoch weniger gut geeignet als das erfindungsgemäß einzusetzende Oxidationsmittel (Sauerstoff). Organische Nitroverbindungen sind jedoch keine geeigneten Oxidationsmittel für das erfindungsgemäße Verfahren.

Das erfindungsgemäße Verfahren wird in Gegenwart von Katalysatorsystemen e) durchgeführt. Diese Katalysatorsysteme enthalten e1) mindestens ein Edelmetall und/oder mindestens eine Edelmetallverbindung der achten Nebengruppe des Periodensystems der Elemente und e2) mindestens eine oxidierend wirkende chinoide Verbindung und/oder mindestens eine Verbindung, welche unter den Reaktionsbedingungen in eine oxidierend wirkende chinoide Verbindung überführt werden kann.

Bei der Katalysatorkomponente e1) handelt es sich entweder um freie Edelmetalle der achten Nebengruppe des Periodensystems oder um Verbindungen dieser Metalle. Besonders vorteilhaft werden die Edelmetalle als im Reaktionsgemisch lösliche Verbindungen zugegeben, beispielsweise Chloride, Bromide, Jodide, Chlorokomplexe, Bromokomplexe, Jodokomplexe, Acetate, Acetylacetonate u. a. lösliche Edelmetallverbindungen. Bevorzugte Edelmetalle sind Palladium, Ruthenium und Rhodium. Besonders bevorzugt wird Palladium, insbesondere in Form von löslichem Palladiumchlorid oder Palladiumacetat eingesetzt.

Bevorzugte Konzentrationen für die Katalysatorkomponente e1) liegem im allgemeinen bei 3 bis 1 000 ppm, vorzugsweise bei 5 bis 100 ppm, berechnet als Edelmetall und auf das gesamte Reaktionsgemisch bezogen, einschließlich des evtl. mitverwendeten Lösungsmittels. Höhere Edelmetall-Konzentrationen sind zwar möglich, wegen der möglichen Edelmetallverluste jedoch unwirtschaftlich, zumal eine weitere Steigerung der Urethanausbeuten nicht mehr erfolgt.

Bei der Katalysatorkomponente e2) handelt es sich um eine oxidierend wirkende chinoide Verbindung und/oder um eine Verbindung, welche unter den Reaktionsbedingungen in eine oxidierend wirkende chinoide Verbindung überführt werden kann. Unter chinoiden Verbindungen sind Verbindungen zu verstehen, wie sie z. B. in « The Chemistry of the Quinoid Compounds » Part I and II (London, Wiley 1974, Herausgeber : Patai) beschrieben werden und wie sie z. B. als Farbstoffe oder Farbstoffvorprodukte vielfach hergestellt werden. Grundsätzlich sind alle derartigen chinoiden Verbindungen als erfindungsgemäße Katalysatorkomponente e2) geeignet, welche unter den erfindungsgemäßen Reaktionsbedingungen dazu befähigt sind, das in der Katalysatorkomponente e1) vorliegende Edelmetall von der Oxidationsstufe Null in eine positive Oxidationsstufe aufzuoxidieren, insbesondere solche, die unter den erfindungsgemäßen Bedingungen dazu befähigt sind, das besonders bevorzugt eingesetzte Palladium von der Oxidationsstufe Null in die Oxidationsstufe + 2 zu überführen.

Anstelle derartiger chinoider Verbindungen können jedoch als erfindungsgemäße Katalysatorkomponente e2) auch in chinoide Verbindungen der genannten Art überführbare Verbindungen eingesetzt werden, d. h. Verbindungen, die durch eine Oxidationsreaktion (hervorgerufen durch die beim erfindungsgemäßen Verfahren mitzuverwendenden Oxidationsmittel d)), Solvolyse oder Eliminierungsreaktion in einer derartige chinoide Verbindung übergehen.

Geeignete chinoide Katalysatorkomponenten e2) sind ortho- und para-Chinone, mehrkernige Chinone und heterocyclische Chinone in substituierter und nichtsubstituierter Form sowie deren Imino-, N-Alkyl- oder N-Aryliminoderivate wie z. B. o-Tetrachlorbenzochinon, p-Tetrachlorbenzochinon, 2,5-Dichlor-3,6-dihydroxy-p-benzochinon, 2-Chlorphenyl-1,4-benzochinon, 2,3-Dichlornaphthochinon, Anthrachinon, 1-Chloranthrachinon, 7-Chlor-4-hydroxy-1.10-anthrachinon, 1-Nitroanthrachinon-2-carbonsäure, 1,5-Dichloranthrachinon, 1,8-Dichloranthrachinon, 2,6-Dichloranthrachinon, 1,4-Dihydroxyanthrachinon, Acenaphthylendion, 5,7-Dichlor-1H-indol-2,3-dion, Indigo oder 1,4-Dihydro-2,3-chinoxalindion.

Auch polymere chinoide Verbindungen, wie sie z. B. von H. G. Cassidy und K. A. Kun in « Oxidation-Reduction Polymers » (Polymer Reviews Vol. 11, Interscience Publ. New York 1965) beschrieben worden sind, sind als Katalysatorkomponente e2) geeignet.

Bevorzugte chinoide Verbindungen sind zur Erhöhung der Oxidationsfähigkeit mit einer oder mehreren elektronenziehenden Substituenten wie z. B. Chlor, Brom, Cyan-, Nitro-, Carbonsäure- oder Sulfonsäuregruppen substituiert. Chinoide Verbindungen, welche N-Aryl- oder N-Alkyl-Substituenten enthalten sind, ebenfalls besonders wirksam. Als Katalysatorkomponente e2) besonders bevorzugte chinoide Verbindungen sind z. B. o-Tetrachlorbenzochinon, p-Tetrachlorbenzochinon, 2,5-Dichlor-3,6-dihydroxy-p-benzochinon, 2,3-Dichlor-1,4-naphthochinon, 7-Chlor-4-hydroxy-1,10-anthrachinon, 1,5-Dichloranthrachinon, 1,8-Dichloranthrachinon und 2-(N-Phenylamino)-3-chlor-1,4-naphthochinon.

Als Katalysatorkomponente e2) geeignete Vorstufen chinoider Verbindungen sind z. B. Ketale der

entsprechenden chinoiden Katalysatorkomponenten sowie hydrierte Formen jener Komponenten, insbesondere die entsprechenden Hydrochinone. Ebenso werden aromatische Amine und mehrkernige Aromaten, die z. B. durch Sulfonsäure-, Carbonsäure-, Nitro- oder Cyangruppen substituiert sind oder die bereits eine Oxogruppe im Ringsystem tragen, unter den oxidativen Reaktionsbedingungen, z. B. durch molekularen Sauerstoff, in erfindungsgemäße chinoide Katalysatorkomponenten e2) übergeführt. Geeignete Vorstufen für chinoide Katalysatorkomponente e2) sind z. B. die Hydrochinone und Ketale der o. g. Chinone oder 5-Amino-2-(phenylamino)-benzolsulfonsäure, 5-Amino-2-((4-chlorphenyl)amino)-benzolsulfonsäure, 4,4'-Diamino-(1,1'-biphenyl)-3,3'-disulfonsäure, 2-Aminobenzolsulfonsäure sowie Benzanthron-3-carbonitril.

Die erfindungsgemäße Katalysatorkomponente e2) wird dem Reaktionssystem im allgemeinen in Konzentrationen von 0,1 Gew.-% bis 5 Gew.-%, bevorzugt in Konzentrationen von 0,5 bis 3 Gew.-%, bezogen auf die Gesamtmenge des Reaktionsgemisches incl. etwa mitverwendeter Lösungsmittel, beigefügt.

Die erfindungsgemäß einzusetzende Katalysatorkomponente e) kann gegebenenfalls als weitere Komponenten bestimmte Metallverbindungen e3) und/oder tert.-Amine e4) enthalten.

Bei der gegebenenfalls mitzuverwendenden Katalysatorkomponente e3) handelt es sich um Magnesiumverbindungen, insbesondere anorganische oder organische Salze des Magnesiums oder um unter den Reaktionsbedingungen zu Redoxreaktionen befähigte Verbindungen von Elementen der dritten bis fünften Hauptgruppe und/oder der ersten bis achten Nebengruppe des Periodensystems der Elemente. Vorzugsweise werden als gegebenenfalls mitzuverwendende Katalysatorkomponente e3) im Reaktionsgemisch zumindest teilweise lösliche Verbindungen der Metalle mit den Ordnungszahlen 12, 22 bis 29, 41, 47, 58 und 92 eingesetzt. Besonders bevorzugte Katalysatorkomponenten e3) sind die Acetate, Nitrate oder Chloride des Chroms, Mangans, Kobalts, Kupfers, Cer oder des Magnesiums, gegebenenfalls in Form der Hydrate oder Aminkomplexe dieser Metallsalze. In Verbindung mit aktivierend wirkenden Chloriden, wie z. B. Ammoniumchloriden, können auch die Oxide der beispielhaft genannten Metalle als Katalysatorkomponenten e3) verwendet werden. Falls an eine Mitverwendung der Katalysatorkomponente e3) gedacht ist, wird diese im allgemeinen in einer ein- bis zehnfach molaren Menge, bezogen auf die Katalysatorkomponente e1), eingesetzt. Dies bedeutet im allgemeinen, daß die Katalysatorkomponente e3) in Mengen von bis zu 0,1 Gew.-%, bezogen auf das Gesamtgewicht des Reaktionsgemischs, incl. gegebenenfalls mitverwendetem Lösungsmittel, mitverwendet werden kann.

Bei der gegebenenfalls mitzuverwendenden Katalysatorkomponente e4) handelt es sich um beliebige tertiäre Amine, welche im Katalysatorsystem die Funktion eines Komplexbildners für die oxidierte Form der Katalysatorkomponente e1) und gegebenenfalls für die Katalysatorkomponente e3) erfüllt, falls die komplexierende Wirkung der im Reaktionsgemisch vorliegenden Ausgangsverbindungen zu diesen Zwecken nicht ausreicht. Prinzipiell sind alle beliebigen tertiären Amine geeignet, d. h. solche, die aliphatisch, cycloaliphatisch, araliphatisch und/oder aromatisch gebundene tertiäre Aminogruppen oder Teile eines heterocyclischen Rings bildende tertiäre Aminogruppen enthalten. Geeignete tertiäre Amine sind beispielsweise Triethylamin, Diisopropylmethylamin, Cyclohexyldiethylamin, Triphenylamin, N,N-Diethylanilin, N-Phenylpiperidin, Pyridin, Chinolin, 1,4-Diaza-(2.2.2.)-bicyclooctan oder Pyrimidin. Bevorzugte tertiäre Amine e4) sind Triethylamin, N,N-Diethylanilin und Pyridin, Die genannten tertiären Amine können auch als Metallsalzkomplexe der Katalysatorkomponente e1) und gegebenenfalls e3) eingesetzt werden. Setzt man die Katalysatorkomponente e1) und/oder gegebenenfalls e3) in oxidischer Form ein, so ist es vorteilhaft, zu deren Aktivierung die tertiären Amine in Form von Hydrochloriden einzusetzen. Die gegebenenfalls zu verwendende Katalysatorkomponente e4) wird in Mengen von bis zu 10 Gew.-% vorzugsweise in 0,2 bis 3 Gew.-%, bezogen auf den Gesamteinsatz der Reaktion, einschließlich des evtl. verwendeten Lösungsmittels eingesetzt. Es ist jedoch auch möglich, die Katalysatorkomponente e4) in höheren Mengen einzusetzen.

Die erfindungsgemäße Umsetzung kann in Gegenwart oder in Abwesenheit eines Lösungsmittels durchgeführt werden. Im allgemeinen dient die vorzugsweise im Überschuß eingesetzte organische Hydroxylverbindung b) als Lösungsmittel. Die Mitverwendung inerter Lösungsmittel, die bis zu 80 Gew.-% des gesamten Reaktionsansatzes ausmachen können, ist jedoch auch möglich. Die Menge des Lösungsmittels muß, gleichgültig, ob es sich um im Überschuß eingesetzte Hydroxylverbindung oder um inertes Lösungsmittel handelt, so bemessen sein, daß die Reaktionswärme der exothermen Urethanbildung ohne unzulässige Temperaturerhöhung abgeführt werden kann. Im allgemeinen wird daher das erfindungsgemäße Verfahren unter Verwendung einer Konzentration an Ausgangsverbindungen a) von 5 bis 30 Gew.-%, vorzugsweise 5 bis 20 Gew.-%, bezogen auf das Gesamtreaktionsgemisch incl. dem. Lösungsmittel, durchgeführt.

Brauchbare Lösungsmittel sind gegenüber den Reaktionskomponenten und dem Katalysatorsystem inerte Lösungsmittel, wie beispielsweise aromatische, cycloaliphatische und aliphatische Kohlenwasserstoffe, die gegebenenfalls halogensubstituiert sein können, wie Benzol, Toluol, Xylol, Chlorbenzol, Dichlorbenzol, Trichlorbenzol, Chlornaphthalin, Cyclohexan, Methylcyclohexan, Chlorcyclohexan, Methylenchlorid, Tetrachlorkohlenstoff, Tetrachlorethan, Trichlortrifluorethan u. ä. Verbindungen sowie tertiäre Amine, wie sie als Katalysatorkomponente e4) beschrieben wurden.

Die Reaktionstemperatur liegt im allgemeinen zwischen 100 und etwa 300 °C, insbesondere zwischen 100 und 250 °C und besonders vorteilhaft im Bereich von 140 bis 220 °C. Der Druck muß so

bemessen sein, daß stets das Vorliegen einer flüssigen Phase gewährleistet ist und liegt im allgemeinen im Bereich von 5 bis 500 bar, besonders vorteilhaft im Bereich von 30 bis 300 bar. Je nach eingesetztem Ausgangsmaterial a) bzw. b) beträgt die für den quantitativen Umsatz benötigte Reaktionszeit zwischen wenigen Minuten und mehreren Stunden.

Das erfindungsgemäße Verfahren kann diskontinuierlich oder kontinuierlich durchgeführt werden. Es ist vorteilhaft, ein Lösungsmittel zu wählen, in welchem das Verfahrensprodukt (Urethan) gut löslich ist. Nach dem Entspannen des Reaktionsmediums und Abkühlen auf 50 °C bis 80 °C fallen die Katalysatorkomponenten e1) bis e3) sowie in komplexierter Form gegebenenfalls e4) in vielen Lösungsmitteln weitgehend bis vollständig aus ; in manchen Fällen ist es vorteilhaft, das Reaktionsgemisch auf 70 bis 50 % des ursprünglichen Volumens einzuengen, um die Katalysatorfällung zu erreichen. Das Katalysatorgemisch kan dann durch Filtrieren oder Zentrifugieren von der urethanhaltigen Lösung abgetrennt werden. Ist das Ausfällen der Katalysatorkomponenten e1) bis e3) nicht möglich, so müssen sie als Rückstand einer z. B. destillativen Aufarbeitung gewonnen werden.

Die nach einer der vorgenannten Methoden abgetrennten Katalysatorkomponenten e1) bis e3) sind in den meisten Fällen rückführbar und wiederum katalytisch aktiv, obwohl sie in chemisch veränderter Form vorliegen. Insbesondere werden beim Einsatz halogenhaltiger Katalysatorkomponenten e2) nach der Reaktion halogenärmere Katalysatorgemische erhalten ; durch Reaktionen mit der Ausgangsverbindung a) können diese dann chemisch gebundenen Stickstoff enthalten.

Je nach Art der Ausgangsstoffe und der Reaktionsbedingungen können durch Nebenreaktionen aus der Ausgangsverbindung a) oder aus dem als Verfahrensprodukt anfallenden Urethan primäre Amine freigesetzt werden. Diese können im allgemeinen zusammen mit dem überschüssigen Ausgangsmaterial b) destillativ aus dem Produktgemisch abgetrennt werden und nach Zufügen von frischen Ausgangsmaterialien a) und b) rückgeführt werden. Solche, durch Nebenreaktionen evtl. entstehenden Amine bilden gemäß DE-OS 3 046 982 mit den gleichen Katalysatorsystemen und unter gleichen Reaktionsbedingungen Urethane, wie sie auch aus den Ausgangsstoffen a) nach dem erfindungsgemäßen Verfahren erhalten werden. Ausbeuteverluste durch Nebenreaktionen sind folglich bei geeigneter Rückführung sehr gering.

Die als Verfahrensprodukte anfallenden Urethane sind im allgemeinen, abgesehen von den Katalysatorkomponenten e1) bis e3), die am schwersten flüchtigen Bestandteile der Produktgemische. Sie können dann nach Abdestillieren der leichter flüchtigen Bestandteile der Produktlösung entweder durch weitere Destillation, welche zumeist im Vakuum durchzuführen ist, oder gegebenenfalls durch Extraktion oder Kristallisation von den Katalysatorkomponenten e1) bis e3) abgetrennt bzw. gereinigt werden. Evtl. verwendete Katalysatorkomponente e4) wird, soweit sie nicht mit den Katalysatorkomponenten e1) und/oder e3) in komplex gebundener Form vorliegt, im allgemeinen gemeinsam mit den leichterflüchtigen Bestandteilen bzw. mit dem Lösungsmittel abdestilliert und rückgeführt.

Die erfindungsgemäßen Verfahrensprodukte (Urethane) eignen sich als Schädlingsbekämpfungsmittel bzw. als Zwischenprodukte zur Herstellung von Schädlingsbekämpfungsmitteln. Sie sind jedoch auch als Ausgangsmaterialien zur Herstellung der ihnen zugrundeliegenden Isocyanate von Interesse. Diese Herstellung der Isocyanate erfolgt durch an sich bekannte thermische Spaltung der erfindungsgemäßen Verfahrensprodukte.

Das Verfahren wird durch die folgenden Beispiele illustriert, ohne damit die Erfindung auf die in den Beispielen gegebenen Bedingungen einzuschränken. Die Mehrzahl der Versuche wurde in einem emaillierten 1,3 l Stahlautoklaven durchgeführt, um katalytische Wandeffekte auszuschließen. Einige Versuche wurden in einem 0,7 l Edelstahlautoklaven durchgeführt. Die Urethanausbeuten beziehen sich jeweils, falls nicht anders angegeben, auf eingesetzten Harnstoff a) gemäß bzw. analog Gl. (2) und sind in Mol-% angegeben. Analog wurden die Ausbeuten an Amin, insbesondere an Anilin (im Falle der Verwendung des besonders bevorzugten Diphenylharnstoffs als Komponente a)), die das Ergebnis von Nebenreaktionen sind, nach der folgenden, rein formalen Gl. (4) berechnet :

Gl. (4) 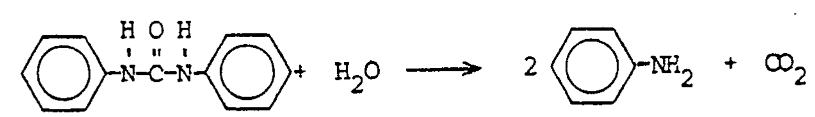

Diese Gleichung ist nur Rechengrundlage und sagt nichts über die tatsächliche Bildungsweise des Amins bzw. Anilins aus.

## Beispiele 1 bis 6

In einem emaillierten 1,3 l Edelstahlautoklaven werden 531,9 g eines Gemisches folgender Zusammensetzung vorgelegt :

94 ppm eines Edelmetallchlorides (s. Tabelle 1), 188 ppm Kupfer-II-acetatmonohydrat, 81,4 Gew.-% Ethanol, 16,9 Gew.-% N,N'-Diphenylharnstoff (DPH) und 1,6 Gew.-% p-Tetrachlorbenzochinon. Bei Raumtemperatur werden sodann 100 bar Kohlenmonoxid und 25 bar Luft aufgepreßt. Unter Rühren wird auf 180 °C aufgeheizt und 1 Stunde bei dieser Temperatur reagieren gelassen.

Nach Abkühlen auf Raumtemperatur wird entspannt und eine zweite gleichartige Reaktionsphase mit frischem CO/Luftgemisch durchgeführt. Es werden somit insgesamt ca. 1,3 Oxidationsäquivalente bezüglich DPH in Form von Luftsauerstoff eingebracht. Aus der gaschromatographischen Analyse werden die in Tabelle 1 angegebenen Ergebnisse erhalten. Beispiel 6 ist ein Vergleichsbeispiel ohne die erfindungsgemäße Katalysatorkomponente e1).

Tabelle 1

| Beispiel Nr. | Katalysator-komponente e1) | Ausbeute in % Phenylurethan | Anilin |
|---|---|---|---|
| 1 | $PdCl_2$ | 77,7 | 10,0 |
| 2 | $RuCl_3$ | 72,2 | 12,4 |
| 3 | $RhCl_3$ | 63,6 | 12,6 |
| 4 | $IrCl_3$ | 56,5 | 17,2 |
| 5 | $PtCl_2$ | 54,9 | 44,0 |
| 6 | - | 48,2 | 47,0 |

Beispiel 7 (Vergleichsbeispiel)

Es wird wie in den Beispielen 1 bis 5 gearbeitet, jedoch ohne die erfindungsgemäßen Katalysatorkomponenten e), d. h. es werden 523 g eines Gemisches, bestehend aus 82,8 Gew.-% Ethanol und 17,2 Gew.-% DPH eingesetzt. Ausbeute : 49,8 % Phenylurethan, 48,2 % Anilin.

Beispiel 8 bis 16

Es wird wie in Beispiel 1 gearbeitet, jedoch werden 532 g eines Gemisches folgender Zusammensetzung eingesetzt : 81,4 Gew.-% Ethanol, 16,9 Gew.-% DPH, 38 ppm Palladiumacetat, 188 ppm Kupfer-II-acetatmonohydrat und 1,6 Gew.-% einer Verbindung entsprechend Katalysatorkomponente e2). In den Beispielen 8 bis 11 ist diese Katalysatorkomponente eine chinoide Verbindung, in den Beispielen 12 bis 16 ist diese Katalysatorkomponente die Vorstufe einer chinoiden Verbindung. Ergebnisse s. Tabelle 2.

Beispiel 17 (Vergleichsbeispiel)

Es wird wie in Beispiel 8 gearbeitet, jedoch ohne die erfindungsgemäßen Katalysatorkomponenten e2) und e3) bzw. e4). Ausbeuten : 40,3 % Phenylurethan und 10,6 % Anilin.

Beispiel 18

Es wird wie in Beispiel 9 gearbeitet, jedoch ohne Zusatz von Kupfer-II-acetatmonohydrat, welches der Katalysatorkomponente e3) entspricht. Ausbeuten : 64,8 % Phenylurethan und 18,8 % Anilin. Das Beispiel zeigt im Vergleich zu Beispiel 9, daß die Katalysatorkomponente e3) im Sinne der Erfindung zwar nicht notwendig ist, daß sie aber zu einer Ausbeuteverbesserung führen kann.

(Siehe Tabelle 2, Seite 9 f.)

Tabelle 2

| Beispiel Nr. | Katalysatorkomponente e2) | Ausbeute in % Phenylurethan | Anilin |
|---|---|---|---|
| 8 | para-Tetrachlorbenzochinon | 83,0 | 10,6 |
| 9 | 2,3-Dichlor-1,4-naphthochinon | 74,0 | 15,2 |
| 10 | 2-Ethyl-9,10-anthrachinon | 58,5 | 40,4 |
| 11 | 9,10-Anthrachinon | 56,2 | 43,0 |
| 12 | Benzanthron-3-carbonitril | 70,5 | 28,8 |
| 13 | 2-Aminobenzolsulfonsäure | 66,5 | 29,2 |
| 14 | 5-Amino-1-hydroxy-2-naphthalinsulfonsäure | 62,1 | 37,6 |
| 15 | 1,2-Dihydro-3,6-pyridazindion | 63,3 | 36,0 |
| 16 | 4,4'-Diamino-(1,1'-biphenyl)-3,3'-disulfonsäure | 63,1 | 36,4 |

Beispiel 19 (Vergleichsbeispiel)

Es wird wie in Beispiel 8 gearbeitet, jedoch wird das Einsatzgemisch nicht mit einem Kohlenmonoxid/Luft-Gemisch, sondern unter 125 bar Stickstoff 2 × 1 Stunde umgesetzt. Ausbeuten : 40 % Phenylurethan und 52 % Anilin.

Beispiel 20

In einem 0,7 l Edelstahlautoklaven werden 286,5 g eines Gemisches folgender Zusammensetzung vorgelegt : 37,6 Gew.-% Ethanol, 43,8 Gew.-% o-Dichlorbenzol, 16,9 Gew.-% DPH, 1,6 Gew.-% 2,3-Dichlor-1,4-naphthochinon, 190 ppm Kupfer-II-acetat-monohydrat und 38 ppm Palladiumacetat. Es wird ein Gasgemisch aus 100 bar CO und 25 bar Luft aufgepreßt, d. h. es werden ca. 0,86 Oxidationsäquivalente bezüglich DPH in Form von Luftsauerstoff eingebracht. Das Reaktionsgemisch wird unter Rühren 1 Stunde bei 180 °C umgesetzt. Ausbeuten : 73 % Phenylurethan, 24 % Anilin. Die Phenylurethanausbeute bezüglich Sauerstoff beträgt ca. 85 %.

Beispiel 21

286,5 g eines Gemisches bestehend aus 81,5 Gew.-% Methanol, 16,9 Gew.-% DPH, 1,6 Gew.-% 2,3-Dichlor-1,4-naphthochinon, 190 ppm Kupfer-II-acetatmonohydrat und 38 ppm Palladiumacetat werden wie in Beispiel 20 angegeben umgesetzt. Ausbeuten : 66 % O-Methyl-N-phenylurethan, 18 % Anilin. Die Urethanausbeute bezüglich Sauerstoff beträgt ca. 100 %.

Beispiel 22

In einem 0,7 l Edelstahlautoklaven werden 263,7 g eines Gemisches folgender Zusammensetzung vorgelegt : 88,5 Gew.-% Phenol, 8,8 Gew.-% N,N'-Dimethylharnstoff (DMH), 2,7 Gew.-% p-Tetrachlorbenzochinon, 180 ppm Kupferacetatmonohydrat und 18 ppm Palladiumacetat. Das Gemisch wird unter Rühren zweimal mit einem Gasgemisch aus 100 bar Kohlenmonoxid und 25 bar Luft jeweils 1 Stunde bei 180 °C umgesetzt. Ausbeute : 38,5 % N-Methyl-O-phenylurethan.

Beispiel 23 (Vergleichsbeispiel)

Dieses Beispiel zeigt im Vergleich zu Beispiel 22, daß eine Umsetzung in Abwesenheit des Oxidationsmittels Luft zwar möglich ist, daß jedoch die Urethanausbeute wesentlich niedriger ist : Es wird wie in Beispiel 22 gearbeitet, jedoch werden anstelle des CO/Luft-Gemisches 125 bar Stickstoff aufgepreßt. Ausbeute : 8 % Urethan.

Beispiel 24

In einem 0,7 l Edelstahlautoklaven werden 284 g eines Gemisches folgender Zusammensetzung vorgelegt : 73,8 Gew.-% o-Dichlorbenzol, 8,2 Gew.-% DMH, 16,4 Gew.-% 2-Propanol, 1,6 Gew.-% p-Tetrachlorbenzochinon, 164 ppm Kupfer-II-acetatmonohydrat und 41 ppm Palladiumacetat. Es wird ein Gasgemisch aus 100 bar CO und 25 bar Luft aufgepreßt, d. h. es werden ca. 0,65 Oxidationsäquivalente bezüglich DMH in Form von Luftsauerstoff eingebracht. Ausbeute : 26 % N-Methylcarbaminsäureisopropylester. Bezüglich Sauerstoff beträgt die Ausbeute ca. 40 %.

Beispiel 25

In einem 0,7 l Edelstahlautoklaven werden 220 g eines Gemisches folgender Zusammensetzung vorgelegt : 91,1 Gew.-% Ethanol, 6,8 Gew.-% Harnstoff, 2,1 Gew.-% 2,3-Dichlor-1,4-naphthochinon, 250 ppm Kupfer-II-acetatmonohydrat und 50 ppm Palladiumacetat. Die Umsetzung erfolgt wie in Beispiel 1 angegeben. Ausbeute : 42 % Carbaminsäureethylester.

Beispiel 26

In einem 0,7 l Edelstahlautoklaven werden 220 g eines Gemisches folgender Zusammensetzung vorgelegt : 87,3 Gew.-% Ethanol, 10,7 Gew.-% N,N,N'-Trimethylharnstoff, 2,0 Gew.-% 2,3-Dichlor-1,4-naphthochinon, 240 ppm Kupfer-II-acetatmonohydrat und 48 ppm Palladiumacetat. Die Umsetzung erfolgt wie in Beispiel 1 angegeben. Ausbeute : 30 % N-Methylcarbaminsäureethylester und 34 % N,N-Dimethylcarbaminsäureethylester. Ca. 60 % des eingesetzten N,N,N'-Trimethylharnstoffs sind nicht umgesetzt.

**Ansprüche**

1. Verfahren zur Herstellung von Urethanen durch Umsetzung von

a) unsubstituierten oder substituierten Harnstoffen, welche jedoch noch mindestens ein, an ein Harnstoff-Stickstoffatom gebundenes Wasserstoffatom aufweisen, mit
b) mindestens eine Hydroxylgruppe enthaltenden organischen Verbindungen und
c) Kohlenmonoxid in Gegenwart von
d) molekularem Sauerstoff und
e) einem mindestens ein Edelmetall und/oder eine Edelmetallverbindung der achten Nebengruppe des Periodensystems der Elemente enthaltenden Katalysatorsystem,

dadurch gekennzeichnet, daß man die Umsetzung in Gegenwart von Katalysatorsystemen e) durchführt, die als weitere Komponente mindestens eine oxidierend wirkende chinoide Verbindung und/oder mindestens eine Verbindung enthalten, welche unter den Reaktionsbedingungen in eine oxidierend wirkende chinoide Verbindung überführt werden kann.

2. Verfahren gemäß Anspruch 1, dadurch gekennzeichnet, daß man als Harnstoff N,N'-Dimethylharnstoff oder N,N'-Diphenylharnstoff einsetzt.

3. Verfahren gemäß Ansprüchen 1 und 2, dadurch gekennzeichnet, daß man als Katalysatorsystem e) ein solches verwendet, welches als weitere Komponente eine Magnesiumverbindung und/oder eine unter den Reaktionsbedingungen zu Redoxreaktionen befähigte Verbindung von Elementen der dritten bis fünften Hauptgruppe und/oder der ersten bis achten Nebengruppe des Periodensystems der Elemente enthält.

4. Verfahren gemäß Ansprüchen 1 bis 3, dadurch gekennzeichnet, daß man als Katalysatorsystem e) ein solches verwendet, welches mindestens ein tertiäres Amin als weitere Komponente enthält.

5. Verfahren gemäß Ansprüchen 1 bis 4, dadurch gekennzeichnet, daß man als Katalysatorsystem e) ein solches verwendet, welches, bezogen auf das Gesamtgewicht des Reaktionsansatzes, 5 ppm bis 100 ppm, berechnet als Edelmetall, mindestens eines Edelmetalls und/oder mindestens einer Edelmetallverbindung enthält.

6. Verfahren gemäß Ansprüchen 1 bis 5, dadurch gekennzeichnet, daß man als Katalysatorsystem e) ein solches verwendet, welches, bezogen auf das Gesamtgewicht des Reaktionsgemischs, 0,1 bis 5 Gew.-% mindestens einer oxidierend wirkenden chinoiden Verbindung und/oder einer Verbindung enthält, welche unter den Reaktionsbedingungen in eine oxidierend wirkende chinoide Verbindung überführt werden kann.

7. Verfahren gemäß Ansprüchen 1 bis 6, dadurch gekennzeichnet, daß man als Katalysatorsystem e) ein solches verwendet, welches, bezogen auf das Gesamtgewicht des Reaktionsgemischs, bis zu 0,1 Gew.-% einer Magnesiumverbindung und/oder bis zu 0,1 Gew.-% einer unter den Reaktionsbedingungen zu Redoxreaktionen befähigten Verbindung von Elementen der dritten bis fünften Hauptgruppe und/oder der ersten bis achten Nebengruppe des Periodensystems der Elemente und/oder bis zu 10 Gew.-% eines tertiären Amins enthält.

8. Verfahren gemäß Ansprüchen 1 bis 7, dadurch gekennzeichnet, daß man die Umsetzung im Druckbereich zwischen 5 und 500 bar und im Temperaturbereich zwischen 100 und 250 °C durchführt.

9. Verfahren gemäß Ansprüchen 1 bis 8, dadurch gekennzeichnet, daß man die Umsetzung, bezogen auf das Gesamtgewicht des Reaktionsgemischs, in Gegenwart von bis zu 80 Gew.-% eines inerten Lösungsmittels durchführt.

10. Verfahren gemäß Ansprüchen 1 bis 9, dadurch gekennzeichnet, daß man die Katalysatorkomponenten e) aus der Lösung des rohen Verfahrensprodukts zurückgewinnt und wiederverwendet.

**Claims**

1. Process for the preparation of urethanes by reacting

a) unsubstituted or substituted ureas which do however still contain at least one hydrogen atom attached to a urea nitrogen atom, with
b) organic compounds containing at least one hydroxyl group and
c) carbon monoxide in the presence of
d) molecular oxygen and
e) a catalyst system containing at least one noble metal and/or noble metal coumpound of the eighth sub-group of the periodic system of elements,

characterised in that the reaction is carried out in the presence of catalyst systems e) which contain as a further component at least one quinoid compound having oxidizing action and/or at least one compound which can be converted, under the reaction conditions, into a quinoid compound having oxidizing action.

2. Process according to Claim 1, characterized in that the urea used is N,N'-dimethylurea or N,N'-diphenylurea.

3. Process according to Claims 1 and 2, characterised in that the catalyst system e) used is one which contains as a further component a magnesium compound and/or a compound of elements of the third to fifth main group and/or of the first to eighth sub-group of the periodic system of elements which is capable of redox reactions under the reaction conditions.

4. Process according to Claims 1 to 3, characterised in that the catalyst system e) used is one which contains at least one tertiary amine as the further component.

5. Process according to Claims 1 to 4, characterised in that the catalyst system e) used is one which, based on the total weight of the reaction mixture, contains 5 ppm to 100 ppm, calculated as noble metal, of at least one noble metal and/or of at least one noble metal compound.

6. Process according to Claims 1 to 5, characterised in that the catalyst system e) used is one which, based on the total weight of the reaction mixture, contains 0.1 to 5 % by weight of at least one compound having oxidizing action and/or of a compound which can be converted, under the reaction conditions, into a quinoid compound having oxidizing action.

7. Process according to Claims 1 to 6, characterised in that the catalyst system e) used is one which, based on the total weight of the reaction mixture, contains up to 0.1 % by weight of a magnesium compound and/or up to 0.1 % by weight of a compound of elements of the third to fifth main group and/or of the first to eigth subgroup of the periodic system of elements which is capable of redox reactions under the reaction conditions, and/or up to 10 % by weight of a tertiary amine.

8. Process according to Claims 1 to 7, characterised in that the reaction is carried out in the pressure range of between 5 and 500 bar and in the temperature range of between 100 and 250 °C.

9. Process according to Claims 1 to 8, characterised in that the reaction is carried out in the presence of up to 80 % by weight, based on the total weight of the reaction mixture, of an inert solvent.

10. Process according to Claims 1 to 9, characterised in that the catalyst components e) are recovered from the solution of the crude product of the process and reused.

**Revendications**

1. Procédé de production d'uréthannes par réaction

a) d'urées non substituées ou substituées qui présentent toutefois encore au moins un atome d'hydrogène lié à un atome d'azote d'urée, avec

b) des composés organiques comportant au moins un groupe hydroxyle et

c) de l'oxyde de carbone en présence

d) d'oxygène moléculaire et

e) d'un système catalyseur comportant au moins un métal noble et/ou un composé de métal noble du huitième sous-groupe du Système Périodique des Eléments,

caractérisé en ce qu'on conduit la réaction en présence de systèmes catalyseurs e) qui comportent comme autre composant au moins un composé quinonique à action oxydante et/ou au moins un composé qui peut être transformé dans les conditions réactionnelles en un composé quinonique à action oxydante.

2. Procédé suivant la revendication 1, caractérisé en ce qu'on utilise comme urée la N,N'-diméthylurée ou la N,N'-diphénylurée.

3. Procédé suivant les revendications 1 et 2, caractérisé en ce qu'on utilise comme système catalyseur e) un système qui comporte comme autre composant un composé de magnésium et/ou un composé, capable de réactions d'oxydo-réduction dans les conditions réactionnelles, d'éléments des troisième à cinquième groupes principaux et/ou des premier à huitième sous-groupes du Système Périodique des Eléments.

4. Procédé suivant les revendications 1 à 3, caractérisé en ce qu'on utilise comme système catalyseur e) un système qui comporte au moins une amine tertiaire comme autre composant.

5. Procédé suivant les revendications 1 à 4, caractérisé en ce qu'on utilise comme système catalyseur e) un système qui comporte, par rapport au poids total de la charge réactionnelle, 5 à 100 ppm, exprimé en métal noble, d'au moins un métal noble et/ou d'au moins un composé de métal noble.

6. Procédé suivant les revendications 1 à 5, caractérisé en ce qu'on utilise comme système catalyseur e) un système qui comprend, par rapport au poids total du mélange réactionnel, 0,1 à 5 % en poids d'au moins un composé quinonique à action oxydante et/ou d'un composé qui peut être transformé dans les conditions réactionnelles en un composé quinonique à action oxydante.

7. Procédé suivant les revendications 1 à 6 caractérisé en ce qu'on utilise comme système catalyseur e) un système qui contient, par rapport au poids total du mélange réactionnel, jusqu'à 0,1 % en poids d'un composé de magnésium et/ou jusqu'à 0,1 % en poids d'un composé, capable de réactions d'oxydo-réduction dans les conditions réactionnelles, d'éléments des troisième à cinquième groupes principaux et/ou des premier à huitième sous-groupes du Système Périodique des Eléments et/ou jusqu'à 10 % en poids d'une amine tertiaire.

8. Procédé suivant les revendications 1 à 7, caractérisé en ce qu'on conduit la réaction dans l'intervalle de pression de 5 à 500 bars et dans l'intervalle de température de 100 à 250 °C.

9. Procédé suivant les revendications 1 à 8, caractérisé en ce qu'on conduit la réaction, par rapport au poids total du mélange réactionnel, en présence d'une proportion allant jusqu'à 80 % en poids d'un solvant inerte.

10. Procédé suivant les revendications 1 à 9, caractérisé en ce qu'on récupère et réutilise les composants e) du catalyseur dans la solution du produit brut du procédé.